(19) Europäisches Patentamt
European Patent Office
Office européen des brevets

(11) **EP 3 251 591 B1**

(12) **FASCICULE DE BREVET EUROPEEN**

(45) Date de publication et mention
de la délivrance du brevet:
**27.02.2019 Bulletin 2019/09**

(51) Int Cl.:
**A61B 5/021** [(2006.01)]    *A61B 5/0215* [(2006.01)]

(21) Numéro de dépôt: **16305641.9**

(22) Date de dépôt: **02.06.2016**

(54) **SYSTÈME DE DÉTERMINATION D'UNE VITESSE D ONDE DE POULS CORONAIRE**

SYSTEM ZUR BESTIMMUNG DER GESCHWINDIGKEIT DER KORONAREN PULSWELLE

SYSTEM FOR DETERMINING A CORONARY PULSE WAVE VELOCITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**06.12.2017 Bulletin 2017/49**

(73) Titulaires:
• **Hospices Civils De Lyon**
  **69229 Lyon Cedex 02 (FR)**
• **Université Claude Bernard Lyon 1**
  **69100 Villeurbanne (FR)**

(72) Inventeurs:
• **LANTELME, Pierre**
  **69270 Fontaines-Saint-Martin (FR)**
• **HARBAOUI, Brahim**
  **69500 Bron (FR)**
• **CIVIDJIAN, Andrei**
  **69330 Meyzieu (FR)**

(74) Mandataire: **GIE Innovation Competence Group**
**310, avenue Berthelot**
**69372 Lyon Cedex 08 (FR)**

(56) Documents cités:
**EP-A1- 1 060 705**

• **TAEWOO NAM ET AL: "A Coronary Pulse Wave Velocity Measurement System", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 août 2007 (2007-08-22), pages 975-977, XP031336335, ISBN: 978-1-4244-0787-3**
• **C. KOLYVA ET AL: "Windkesselness of coronary arteries hampers assessment of human coronary wave speed by single-point technique", AMERICAN JOURNAL OF PHYSIOLOGY: HEART AND CIRCULATORY PHYSIOLOGY, vol. 295, no. 2, 27 juin 2008 (2008-06-27) , pages H482-H490, XP055325584, US ISSN: 0363-6135, DOI: 10.1152/ajpheart.00223.2008**
• **SUYOUNG BANG ET AL: "A pulse transit time measurement method based on electrocardiography and bioimpedance", BIOMEDICAL CIRCUITS AND SYSTEMS CONFERENCE, 2009. BIOCAS 2009. IEEE, IEEE, PISCATAWAY, NJ, USA, 26 novembre 2009 (2009-11-26), pages 153-156, XP031594985, ISBN: 978-1-4244-4917-0**

**Description**

L'invention concerne les systèmes d'assistance en vue d'étudier des pathologies cardiaques, et en particulier les systèmes permettant d'anticiper un risque de rupture d'une plaque intra coronaire, en vue d'affiner la stratégie de prise en charge d'un patient.

Il est connu que les calcifications d'une artère étaient à l'origine d'une rigidification de cette artère. La rigidité vasculaire est reconnue comme jouant un rôle important dans les accidents vasculaires. Des études médicales ont suggéré que la vitesse d'onde de pouls au niveau d'un segment artériel était assez représentative d'une telle rigidité. Des études ont également confirmé que la rigidité aortique, mesuré par sa vitesse d'onde de pouls, était également un indicateur permettant d'anticiper des pathologies coronaires.

S'il existe des systèmes permettant de réaliser des mesures de vitesses d'onde de pouls aortiques, et donc de réaliser des études correspondantes, il n'existe pas de système permettant de déterminer avec précision une vitesse d'onde de pouls coronaire. Les praticiens ne disposent donc d'aucun système leur permettant de mesurer la vitesse d'onde de pouls coronaire et de déterminer ainsi l'impact de la rigidité coronaire sur l'évolution d'une lésion coronaire, comme par exemple le risque de thrombose aigue. Par ailleurs, la détermination de la vitesse d'onde de pouls aortique s'avère insuffisante pour déterminer avec précision les pathologies sur les artères coronaires. En particulier, la mesure de la vitesse d'onde de pouls aortiques ne permet pas d'anticiper un risque de rupture d'une plaque intra coronaire.

Le calcul de la vitesse d'onde de pouls coronaire est actuellement uniquement basé sur des calculs manuels, à titre expérimental. En particulier, le document 'A Coronary Pulse Wave Velocity Measurement System', publié par Messieurs Taewoo Nam et alias, pages 975 à 977 dans Proceedings of the 29th Annual International Conférence of the IEEE EMBS, dans le cadre d'une conférence à la Cité Internationale de Lyon en France du 23 au 26 Août 2007, décrit un tel procédé.

Selon ce procédé, une sonde de pression est placée dans une position proximale dans une artère coronaire pour mesurer la pression sanguine à cet emplacement sur au moins une pulsation cardiaque. La sonde de pression est ensuite déplacée d'une distance prédéfinie vers une position distale dans l'artère coronaire pour mesurer la pression sanguine à cet emplacement sur au moins une pulsation cardiaque. Un signal d'électrocardiogramme est mesuré simultanément à ces signaux de pression.

Par une analyse manuelle, un analyste identifie un front montant de la pression sanguine proximale, puis il identifie un front montant de la pression sanguine distale. L'identification de chaque front montant se fait par recherche de l'intersection entre la tangente à ce front montant de pression et une droite horizontale passant au niveau de la pression diastolique. A partir du délai relatif des signaux de pression sanguine en positions distale et proximale par rapport au complexe QRS du signal d'électrocardiogramme, l'analyste détermine la durée séparant ces deux fronts montants. L'analyste calcule ensuite la vitesse de l'onde de pouls coronaire en divisant la distance prédéfinie par la durée calculée entre les fronts montants.

On constate que dans environ 50% des échantillons de mesures de pression distale, un artefact de montée de pression intervient probablement avant l'arrivée du front d'onde effectif de la pulsation cardiaque. Dans ce cas, l'application de cette méthode connue de calcul de la vitesse de l'onde de pouls coronaire donne des résultats aberrants. Par ailleurs, à cause de phénomènes de microcirculation sanguine, la valeur de la pression sanguine diastolique peut être altérée, faisant apparaître une onde pré-systolique, comme mentionné dans l'article intitulé 'Alterations of pressure waveforms along the coronary arteries and the effect of microcirculatory vasodilation', publié par Mrs Vavuranakis et alias, dans International Journal of Cardiology 117 en 2007, aux pages 254-259. Par conséquent, le front montant de la pression distale, calculé par l'intersection entre la tangente entre la montée de pression et la valeur diastolique, est déterminé de façon imprécise. Par conséquent, une telle analyse n'est pas applicable pour une utilisation en routine médicale.

D'autre part, le caractère ischémiant d'une sténose coronaire est à présent estimé à l'aide de la technique appelée FFR selon la procédure suivante. Un filament-guide muni au bout d'un capteur de pression artérielle est inséré dans l'artère coronaire en position proximale puis en position distale et des mesures de pression artérielle sont effectuées à travers ce filament guide dans chaque position en situation basale puis après injection d'adénosine. L'adénosine est un vasodilatateur administré par voie veineuse ou intra-coronaire afin de provoquer une hyperhémie maximale provoquant un afflux de sang semblable à celui provoqué par un effort physique.

Le rapport entre les pressions artérielles distales et proximales est calculé, ce rapport étant un reflet du caractère ischémiant d'une sténose coronaire: un rapport inférieur à 0.8 signifie qu'il existe une ischémie significative nécessitant un traitement. Si le rapport est supérieur à 0.8, la sténose n'est pas considérée comme ischémmiante et ne sera pas traitée par voie invasive. Ce seuil de 0,8 pourrait en théorie varier en fonction des caractéristiques biomécaniques de l'artère.

La FFR est capable de donner une information sur le caractère ischemiant d'une sténose coronaire mais pas sur son potentiel d'évolution vers une rupture de plaque. En effet, une lésion coronaire non ischémiante ne signifie pas qu'elle ne peut pas être à l'origine d'un accident aigu responsable d'infarctus du myocarde, la rupture de plaque que la FFR est incapable de prédire. Ces plaques vulnérables sont difficiles à détecter et elles auraient des caractéristiques biomécaniques particulières en étant plus élastiques.

La détermination des caractéristiques biomécaniques des artères coronaires ne se fait pas en pratique clinique, elle pourrait pourtant avoir des implications majeures. La détermination de la vitesse de l'onde de pouls coronaire est un reflet direct des caractéristiques biomécaniques des artères coronaires.

Toutefois, l'injection d'adénosine est à risque pour le patient car cela induit un ralentissement du rythme cardiaque pouvant aller jusqu'à l'arrêt cardiaque. De plus, cela prend du temps, ralentissant la procédure. De ce fait, une méthode d'estimation du caractère ischémiant d'une sténose coronaire est souhaitable sans avoir recours à des substances vasodilatatrices comme l'adénosine.

L'invention vise également à résoudre un ou plusieurs de ces inconvénients.

L'invention porte ainsi sur un système de détermination d'une vitesse d'onde de pouls coronaire, comprenant :

- une interface de réception d'un signal de pression sanguine proximale dans une artère coronaire et de réception d'un signal de pression sanguine distale dans cette artère coronaire;
- un dispositif de traitement, configuré pour :

  - identifier un instant Tfmp du début d'un front montant et un instant Tfdp du début d'un front descendant à partir d'un signal de pression proximale reçu ;
  - calculer la durée T entre les instants Tfdp et Tfmp identifiés ;
  - identifier un instant Tfdd du début du front descendant à partir d'un signal de pression distale reçu ;
  - identifier un instant Tfmd du début du front montant du signal de pression distale, après avoir retranché ladite durée calculée T de l'instant Tfdd du début du front descendant du signal de pression distale ;
  - récupérer la valeur de la distance entre le lieu de mesure de la pression proximale et le lieu de mesure de la pression distale ;
  - calculer la durée séparant le début des fronts montants identifiés respectifs du signal de pression proximale et du signal de pression distale ;
  - calculer la vitesse d'onde de pouls coronaire en fonction de ladite durée séparant les fronts montants et de ladite valeur de distance récupérée.

L'invention porte également sur les variantes suivantes. L'homme du métier comprendra que chacune des caractéristiques des variantes suivantes peut être combinée indépendamment aux caractéristiques ci-dessus, sans pour autant constituer une généralisation intermédiaire.

Selon une variante, ledit dispositif de traitement est configuré pour calculer la dérivée du signal de pression sanguine distale, et configuré pour identifier Tfmd comme correspondant à l'instant du pic de la dérivée calculée le plus proche de l'instant défini par Tfdd-T.

Selon une variante, ledit dispositif de traitement est configuré pour :

- dériver un signal de pression sanguine distale $PSCD_2$ par soustraction d'une pression diastolique distale du signal de pression sanguine distale reçu ;
- dériver un signal de pression sanguine proximale $PSCP_2$ par soustraction d'une pression diastolique proximale du signal de pression sanguine proximale reçu ;
- synchroniser les signaux $PSCD_2$ et $PSCP_2$ par le début de leurs fronts descendants respectifs;
- calculer une mesure de ressemblance entre les signaux $PSCD_2$ et $PSCP_2$ sur la durée T.

Selon encore une variante, ledit dispositif de traitement est configuré pour déterminer Tfmd dans un intervalle défini selon l'inégalité suivante :

$$Tfdd-1,1*T< Tfmd < Tfdd -0,9*T.$$

Selon encore une autre variante, le dispositif de traitement détermine l'instant Tfmd à l'intersection entre une pression diastolique distale et une tangente à un front montant d'un signal de pression distale.

Selon une variante, l'interface de réception est configurée pour recevoir un signal d'électrocardiogramme, et le dispositif de traitement est configuré pour calculer les durées séparant un complexe QRS du signal d'électrocardiogramme respectivement de l'instant Tfmp et de l'instant Tfmd.

Selon encore une variante, le dispositif de traitement détermine ladite pression diastolique distale en calculant l'intersection entre :

- une fonction polynomiale ou logarithmique ajustée au signal de pression distale avant l'instant du début du complexe QRS et extrapolée après l'instant du début du complexe QRS ; et
- la tangente au front montant de la pression distale.

Selon une autre variante, le dispositif de traitement détermine l'instant Tfmp à l'intersection entre une tangente au front montant du signal de pression proximale et une pression diastolique proximale, et dans lequel le dispositif de traitement détermine l'instant Tfdp à l'intersection entre une tangente au front descendant du signal de pression proximale et une pression systolique proximale.

Selon encore une variante, le dispositif de traitement est configuré pour calculer les durées séparant un complexe QRS du signal d'électrocardiogramme respectivement de l'instant Tfmp et de l'instant Tfmd en:

- créant un premier signal recomposé égal à zéro partout sauf au cours d'une période d'une largeur de 10-20 ms et centrée sur l'instant correspondant au front montant de la pression sanguine Tfmp au cours de laquelle ledit signal recomposé est égal à une constante, à une fonction triangulaire ou à l'amplitude du complexe QRS du signal d'électrocardiogramme,
- créant un deuxième signal recomposé égal à zéro partout sauf au cours d'une période d'une largeur de 10-20 ms et centrée sur l'instant correspondant au front montant de la pression sanguine Tfmd au cours de laquelle ledit signal recomposé est égal à une constante, à une fonction triangulaire ou à l'amplitude du complexe QRS du signal d'électrocardiogramme,
- calculant la fonction d'inter-corrélation entre le signal de l'électrocardiogramme et le premier signal recomposé sur la durée d'un cycle cardiaque ou sur la durée d'un cycle respiratoire ;
- calculant la fonction d'inter-corrélation entre le signal de l'électrocardiogramme et le deuxième signal recomposé sur la durée d'un cycle cardiaque ou sur la durée d'un cycle respiratoire ;
- calculant la moyenne sur un cycle cardiaque ou respiratoire de ladite durée séparant un complexe QRS du signal d'électrocardiogramme respectivement de l'instant Tfmp et de l'instant Tfmd, à partir du décalage entre l'électrocardiogramme et les signaux recomposés pour lesquels la fonction d'inter-corrélation est maximum, si et seulement si la valeur de ce maximum est au-dessus d'un seuil compris de préférence entre 0.4 et 0.6.

Selon encore une autre variante, si le calcul de la mesure de ressemblance entre les signaux $PSCD_2$ et $PSCP_2$ sur la durée T est insuffisante, le dispositif de traitement calcule une mesure de ressemblance entre les signaux $PSCD_2$ et $PSCP_2$ sur un intervalle compris entre $0,2*T$ et $0,8*T$ des signaux synchronisés, avec différentes valeurs de décalages temporels, détermine pour quel décalage temporel Tajust cette mesure de ressemblance entre les signaux $PSCD_2$ et $PSCP_2$ sur cet intervalle est maximale, détermine Tfmd = Tfdd-T+Tajust.

Selon une variante, si le calcul de la mesure de ressemblance entre les signaux $PSCD_2$ et $PSCP_2$ sur la durée T est insuffisante, le dispositif de traitement

- récupère un signal de pression sanguine proximale entre les instants Tfmp et Tfdp ;
- adapte le signal récupéré à l'échelle du signal de pression sanguine distale ;
- remplace le signal de pression sanguine distale entre les instants Tfdd - T et Tfdd par le signal de pression récupéré et adapté;
- détermine Tfmd comme l'intersection entre la tangente au signal récupéré adapté et une pression diastolique distale.

Selon une autre variante, le dispositif de traitement :

- détermine la valeur du signal de pression sanguine distale à l'instant Tpfm=Tfmd-Tana, avec Tana compris entre 5 et 10ms ;
- calcule le rapport entre ladite valeur de pression sanguine déterminée à l'instant Tpfm et une pression sanguine diastolique distale ;
- génère un signal d'alerte si le rapport calculé est supérieur à un seuil compris entre 0.95 et 1.05.

Selon encore une variante, le système comprend en outre un fil-guide allongé de FFR, comportant deux capteurs de pression décalés d'une distance prédéfinie sur la longueur du fil-guide, lesdits deux capteurs de pression étant connectés à ladite interface de réception, ladite interface de réception comprenant un circuit d'échantillonnage des signaux respectifs desdits capteurs de pression.

Selon une variante, ladite distance prédéfinie entre lesdits deux capteurs de pression est comprise entre 70 et 150mm.

D'autres caractéristiques et avantages de l'invention ressortiront clairement de la description qui en est faite ci-après, à titre indicatif et nullement limitatif, en référence aux dessins annexés, dans lesquels :

- la figure 1 est une représentation schématique d'un coeur et de ses artères coronaires ;
- la figure 2 est une vue en coupe d'un fil-guide selon un aspect de l'invention, inséré dans une artère coronaire comportant une sténose ;
- la figure 3 est une vue en coupe schématique d'un dispositif à fil-guide de FFR selon un aspect de l'invention ;
- la figure 4 est une représentation schématique d'un système de traitement de signaux en vue de déterminer la vitesse d'onde de pouls et le caractère ischémiant d'une sténose coronaire selon un aspect de l'invention ;
- la figure 5 est un diagramme illustrant un exemple de pression sanguine coronaire proximale PSCP, avec un signal d'électrocardiogramme correspondant, la dérivée de cette pression PSCP, et un signal recomposé à partir du signal d'électrocardiogramme (SPR), comme détaillé par la suite ;
- la figure 6 est un diagramme illustrant un exemple de pression sanguine coronaire distale PSCD, avec un signal d'électrocardiogramme correspondant, la dérivée de cette pression PSCD, et un signal recomposé à partir du signal d'électrocardiogramme (SDR), comme détaillé par la suite ;
- la figure 7 illustre un exemple de traitement réalisé sur le signal de pression PSCP ;
- la figure 8 illustre un exemple de traitement réalisé sur le signal de pression PSCD ;
- les figures 9 et 10 illustrent la sélection d'une portion du signal PSCP ;
- les figures 11 et 12 illustrent l'utilisation d'une portion du signal PSCP pour déterminer un front montant du signal PSCD ;
- la figure 13 est un détail d'un diagramme de pression sanguine coronaire distale PSCD illustrant un cas particulier de détermination de pression diastolique extrapolée (PDE).

L'invention permet de déterminer précisément et de façon reproductible la vitesse d'onde de pouls coronaire, (et éventuellement le degré de sténose de la lésion coronaire) facilitant ainsi une prise de décision du praticien, en vue de déterminer la prise en charge du patient. De plus, l'invention peut être mise en oeuvre en même temps que la procédure d'introduction d'un fil-guide de FFR déjà validée cliniquement.

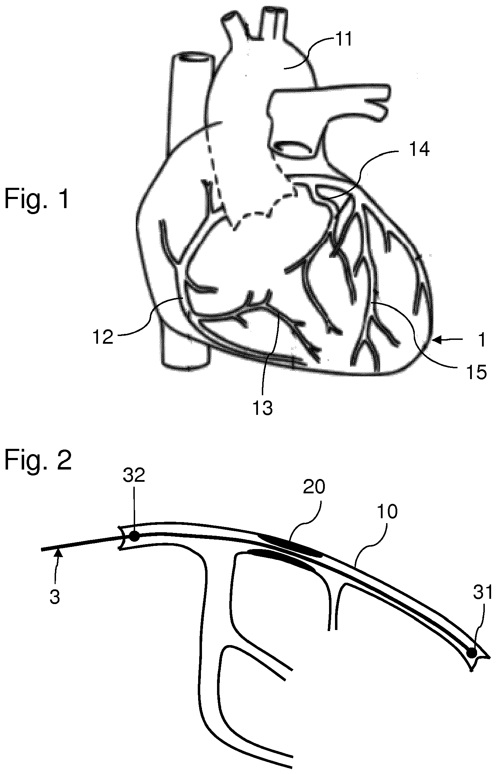

La figure 1 est une représentation schématique d'un coeur humain 1. On distingue l'artère aorte 11 raccordée au coeur, et des artères coronaires 12 à 15. Les artères coronaires sont destinées à alimenter les muscles cardiaques en sang oxygéné. La figure 1 illustre notamment l'artère coronaire droite 12, l'artère coronaire postérieure descendante 13, l'artère coronaire circonflexe gauche 14 et l'artère coronaire antérieure gauche descendante 15.

La figure 2 permet d'illustrer un exemple d'un mode de récupération de signaux, en vue de calculer la vitesse d'onde de pouls coronaire d'un patient. Un fil-guide de FFR 3 est introduit de façon à positionner son extrémité libre à l'intérieur d'une artère coronaire 10. Le fil-guide 3 comporte ici deux capteurs de pression 31 et 32 au niveau de son extrémité libre. Le capteur de pression 31 est en position distale, afin de mesurer la pression sanguine à proximité de l'embranchement de l'artère 10 avec le coeur. Le capteur de pression 32 est en position proximale, afin de mesurer la pression sanguine à proximité de l'embranchement de l'artère 10 avec l'artère aortique. Le capteur de pression 32 est décalé par rapport au capteur de pression 31, d'une distance Dmd prédéfinie sur la longueur du fil-guide 3. L'artère coronaire 10 illustrée ici comporte une sténose 20, les capteurs de pression 31 et 32 étant positionnés de part et d'autre de cette sténose 20.

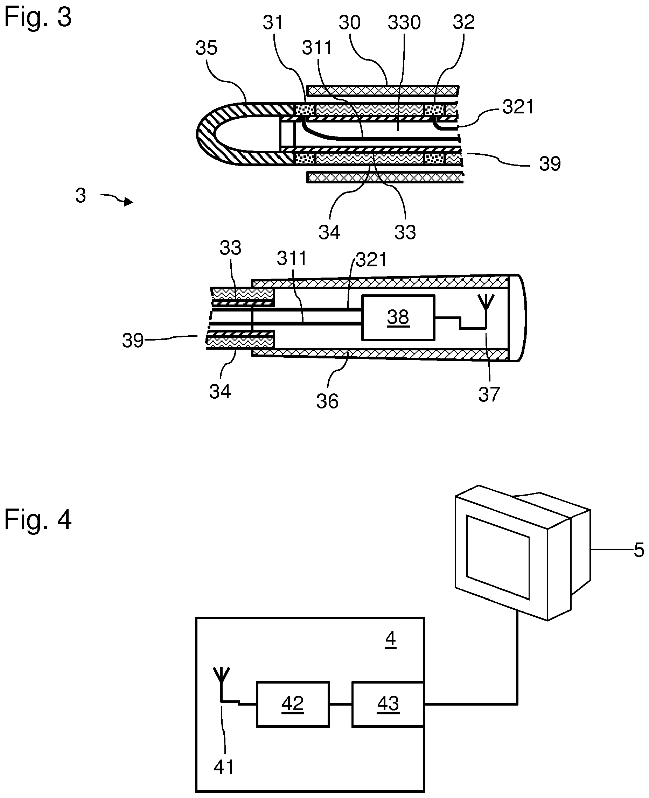

La figure 3 est une vue en coupe schématique de deux extrémités d'un fil-guide 3 selon un aspect de l'invention. Le fil-guide 3 comporte un fil 39 coulissant de façon connue en soi dans une gaine externe de stockage 30. Le fil 39 est illustré uniquement de façon schématique pour détailler sa structure, le fil 39 n'est pas illustré à l'échelle. Le fil 39 est flexible pour s'adapter à la morphologie de l'artère coronaire dans lequel il est introduit. Le fil 39 comporte un fourreau métallique creux 33. Le fourreau métallique 33 est recouvert d'une gaine 34 en matériau synthétique. Le fil 39 comporte avantageusement un embout 35 au niveau de son extrémité libre. L'embout 35 peut avantageusement être flexible et radio-opaque. L'embout 35 est ici fixé sur le fourreau métallique 33.

Le capteur de pression 31 est ici fixé à la périphérie du fourreau 33, et positionné entre l'embout 35 et la gaine 34. Le capteur de pression 31 est destiné à mesurer la pression sanguine distale. Le capteur de pression 31 (de structure connue en soi) est connecté à un câble 311 de transmission du signal de pression. Le câble 311 traverse un orifice ménagé dans le fourreau 33 pour sa connexion au capteur 31. Le fil 311 s'étend dans un alésage interne 330 du fourreau 33.

Le capteur de pression 32 est ici fixé à la périphérie du fourreau 33, et positionné entre deux tronçons de la gaine 34. Le capteur de pression 32 est destiné à mesurer la pression sanguine proximale. Le capteur de pression 32 est connecté à un câble 321 de transmission du signal de pression. Le câble 321 traverse un orifice ménagé dans le fourreau 33 pour sa connexion au capteur 32. Le fil 321 s'étend dans l'alésage interne 330 du fourreau 33.

Le fil 39 est flexible mais sensiblement non compressible ou inextensible. Ainsi, le fil 39 maintient une distance Dmd constante entre les capteurs 31 et 32. La distance entre les capteurs 31 et 32 correspond en pratique à la distance curviligne entre ces capteurs le long du fil 39. La distance entre les capteurs 31 et 32 est avantageusement au moins égale à 70 mm, de façon à garantir une distance suffisamment élevée entre ces capteurs 31 et 32, afin de disposer d'une bonne précision pour le calcul de la vitesse d'onde de pouls. Par ailleurs, la distance entre les capteurs 31 et 32 est avantageusement au plus égale à 150 mm, de sorte que le fil-guide 3 reste utilisable dans la plupart des artères coronaires de longueur standard. Par ailleurs, l'utilisation d'un fil-guide 3 comportant des capteurs 31 et 32 maintenus à une distance prédéfinie permet d'éliminer les imprécisions liées à la distance entre deux mesures de pression à l'intérieur d'une artère coronaire.

À l'opposée de son extrémité libre, le fil 39 est fixé à une poignée de préhension 36. Le fourreau 33 et la gaine 34 sont ici encastrés dans la poignée de préhension 36. La poignée 36 permet ainsi de déplacer le fil 39. Dans cet exemple, le fil-guide 3 est configuré pour fournir les signaux de pression mesurés à un système de traitement, par l'intermédiaire d'une interface sans fil. On peut cependant également envisager que le fil-guide 3 communique avec un système de traitement par une interface câblée. Un circuit de numérisation et de pilotage 38 est ici logé à l'intérieur de la poignée 36. Les câbles 311 et 321 du fil 39 sont connectés au circuit 38. Le circuit 38 est connecté à une antenne émettrice 37. Le circuit 38 est configuré pour numériser les signaux mesurés par les capteurs 31 et 32 et fournis par les câbles 311 et 321. Le circuit 38 est également configuré pour transmettre les signaux numérisés à distance, par l'intermédiaire de l'antenne 37, par un protocole de communication approprié. L'alimentation électrique du circuit 38 se fait de façon connue en soi et ne sera pas décrite ici.

La gaine 34 peut être réalisée en matériau hydrophobe au niveau de l'extrémité libre du fil 39, et peut être réalisée dans un autre matériau tel que du PTFE entre l'extrémité libre et la poignée 36.

Le fil-guide 3 communique avec un système de traitement de signaux 4. Le système 4 comprend ici une interface de communication 41 avec le fil-guide 3 de type sans fil. Le système 4 comprend ainsi une antenne de réception 41, configurée pour recevoir les informations communiquées par l'antenne 37. L'antenne de réception 41 est connectée à un circuit de traitement 42. Le système 4 comprend une interface de communication filaire 43. L'interface 43 permet par exemple d'afficher les résultats calculés par le circuit de traitement 42 sur un écran d'affichage 5. Un convertisseur analogique/numérique peut par exemple être intégré dans le circuit de traitement 42, soit dans le fil-guide 3, afin de permettre au circuit de traitement 42 de réaliser des traitements sur des signaux numériques de pressions sanguines coronaires proximale et distale.

Le circuit de traitement 42 est configuré pour réaliser les traitements suivants :

- identifier un instant Tfmp du début d'un front montant dans le signal de pression sanguine coronaire proximale (désignée par PSCP par la suite) reçu sur l'interface 41 ;
- identifier un instant Tfdp du début d'un front descendant dans le signal de pression PSCP reçu sur l'interface 41 ;
- calculer la durée T entre les instants Tfmp et Tfdp qui ont été identifiés ;
- identifier un instant Tfdd du début d'un front descendant dans le signal de pression sanguine coronaire distale (désignée par PSCD) reçu sur l'interface 41 ;
- identifier un instant Tfmd du début d'un front montant dans le signal de pression PSCD reçu sur l'interface 41, en retranchant la durée T de l'instant Tfdd. On sélectionnera par exemple un instant Tfmd tel que Tfdd -1,1*T < Tfmd < Tfdd -0,9*T ;
- récupérer la valeur de la distance Dmd entre le lieu de mesure de la pression proximale et le lieu de mesure de la pression distale. Cette distance Dmd est par exemple mémorisée dans le fil-guide 3 ou dans le système 4 lorsque le fil-guide 3 comporte deux capteurs de pression 31 et 32 espacés d'une distance prédéterminée ;
- calculer la durée Tmd séparant le début des fronts montants Tfmp et Tfmd ;
- calculer la vitesse d'onde de pouls coronaire à partir de cette durée Tmd et de la distance Dmd.

Avantageusement, on dérive un signal $PSCD_2$ en retranchant la pression diastolique distale du signal PSCD (la pression diastolique distale sera détaillée par la suite), on dérive un signal $PSCP_2$ en retranchant la pression diastolique proximale du signal PSCP (la pression diastolique proximale sera détaillée par la suite), on synchronise les signaux $PSCP_2$ et $PSCD_2$ par les débuts de leurs fronts descendants, puis on calcule une mesure de ressemblance entre les signaux $PSCP_2$ et $PSCD_2$ pour une durée égale à T. La mesure de ressemblance peut être calculée avec un écart quadratique moyen entre les signaux $PSCP_2$ et $PSCD_2$ ou par une fonction d'auto-corrélation entre ces signaux. On répète le calcul de la mesure de ressemblance entre les signaux $PSCP_2$ et $PSCD_2$ pour une durée égale à T et pour plusieurs décalages entre $PSCP_2$ et $PSCD_2$ dans l'intervalle de temps [-0.1 * T; 0.1 * T], avec des incréments temporels par exemple entre 0.1ms et 0.5ms (correspondant à la période d'échantillonnage), sur une fenêtre de +/-20ms. Le décalage optimum pour une meilleure ressemblance entre $PSCP_2$ et $PSCD_2$ (Tajust) est calculé.

On réalise ensuite les étapes d'identification de l'instant Tfmd par retrait de la durée T de l'instant Tfdd et par le rajout de Tajust, on calcule la durée Tmd, on récupère la valeur de la distance Dmd, et on calcule la vitesse d'onde de pouls coronaire comme détaillé auparavant,en divisant Dmd par Tmd..

De façon pratique, on peut constater que le signal de pression PSCP est très fidèle à la pression aortique. Le signal de pression PSCP comporte ainsi une quantité réduite d'artefacts pouvant altérer le résultat de traitements numériques auxquels il est soumis. A contrario, notamment du fait de phénomènes de microcirculation sanguine, le signal de pression PSCD peut comporter une quantité importante d'artefacts pouvant altérer le résultat de traitements numériques auquel il est soumis. En particulier, le front montant du signal de pression PSCD s'est avéré particulièrement sensible à de tels artefacts, la détermination directe du début de ce front montant s'avérant particulièrement imprécise par une analyse de ce seul front montant. A contrario, les inventeurs ont constaté que le front descendant du signal de pression PSCD était peu impacté par des artefacts. Par conséquent, l'invention propose de déterminer le début du front montant du signal de pression PSCD (ou $PSCD_2$ ) en déterminant le début du front descendant du signal de pression PSCD, puis en remontant à partir de là au début du front montant du signal de pression PSCD à partir de la durée séparant les fronts montants et descendants déterminés précisément pour le signal de pression PSCP (ou $PSCP_2$).

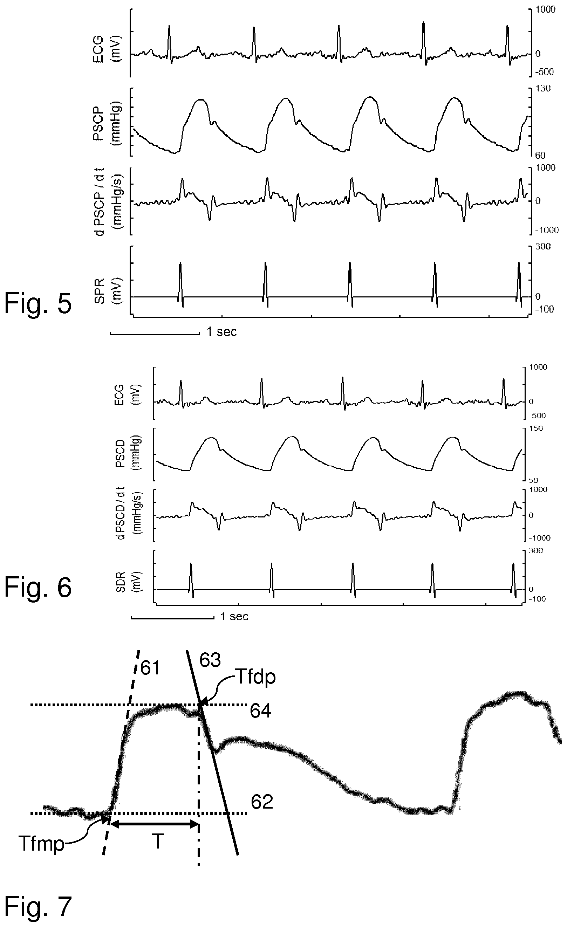

La figure 7 illustre un exemple de traitement réalisé sur le signal de pression PSCP. Dans cet exemple, le circuit de traitement 42 détermine :

- la tangente 61 (illustrée en trait discontinu) au front montant de pression PSCP ;
- la pression diastolique illustrée par la droite 62 (illustrée en pointillés) ;
- la tangente 63 (illustrée en trait continu) au front descendant de pression PSCP ;
- la pression systolique illustrée par la droite 64 (illustrée en pointillés).

Le circuit de traitement 42 détermine ici l'instant de début Tfmp du front montant de la pression PSCP par l'abscisse de l'intersection entre la tangente 61 et la droite de pression diastolique 62. Le circuit de traitement 42 détermine ici l'instant de début Tfdp du front montant de la pression PSCP par l'abscisse de l'intersection entre la tangente 63 et la droite de pression systolique 64.

D'autres procédés de détermination des instants Tfmp et/ou Tfdp peuvent également être mis en oeuvre par le circuit de traitement 42. On peut par exemple calculer la dérivée première ou deuxième de la pression PSCP, puis déterminer les instants auxquels cette dérivée première ou deuxième franchit un seuil positif et un seuil négatif respectivement (pour identifier respectivement un pic positif et un pic négatif). Ces instants peuvent être utilisés respectivement pour les valeurs Tfmp et Tfdp.

La pression systolique et la pression diastolique peuvent être calculées de façon connue en soi, en analysant la pression PSCP sur au moins une pulsation cardiaque complète, en prenant par exemple les extremums de la pression PSCP.

Avantageusement, préalablement au traitement des signaux PSCP (ou $PSCP_2$) ou PSCD (ou $PSCD_2$), le circuit 42 peut mettre en oeuvre un filtrage passe-bas (par exemple avec une fréquence de coupure entre 10 et 20 Hz), pour éliminer les fluctuations rapides de pression entre les battements cardiaques.

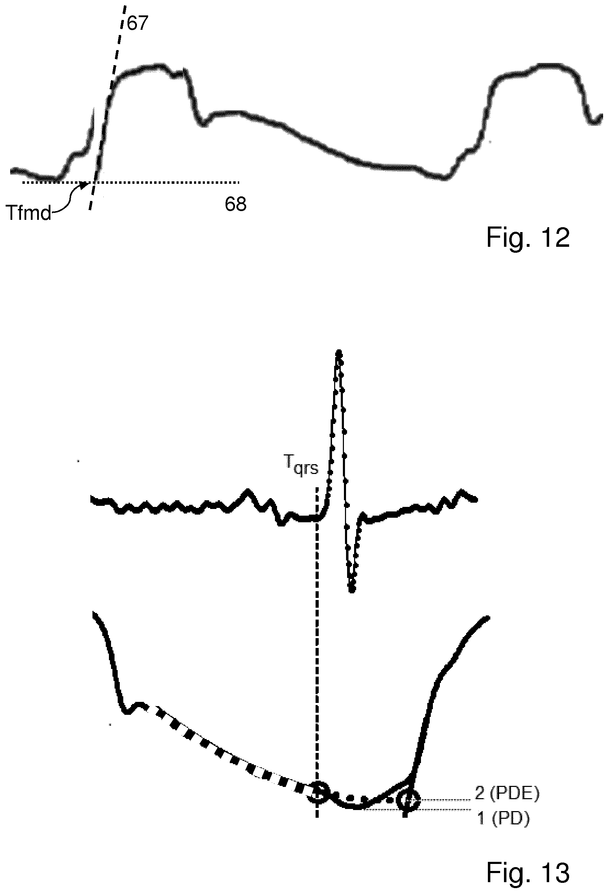

Avantageusement, comme illustré à la figure 13 pour la pression sanguine distale (applicable également à la pression sanguine proximale), la pression diastolique utilisée dans les calculs sera déterminée par une extrapolation :

- on détermine l'instant Tqrs juste avant la contraction du coeur (complexe QRS) ;
- on détermine une fonction polynomiale ou logarithmique ajustée sur les échantillons de la pression sanguine avant Tqrs (trait grossi)
- on détermine la valeur de la pression diastolique distale en extrapolant la fonction polynomiale ou logarithmique ajustée précédemment à l'instant Tfmp ou Tfdd-T+Tajust (pointillés carrés). Cette valeur est définie comme valeur de pression diastolique extrapolée (PDE).

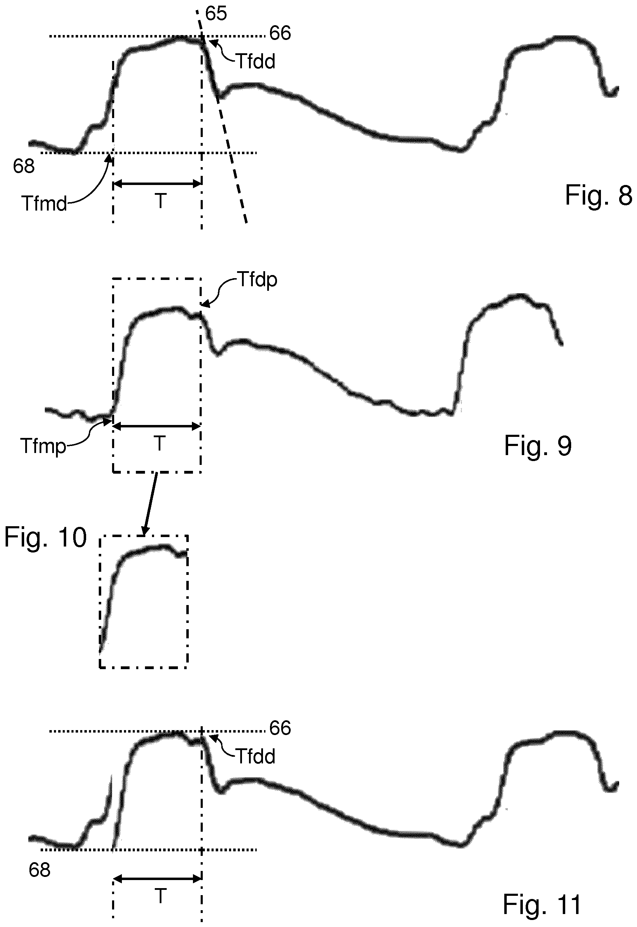

La figure 8 illustre un exemple de traitement réalisé sur le signal de pression PSCD. Dans cet exemple, le circuit de traitement 42 détermine :

- la tangente 65 (illustrée en trait discontinu) au front descendant de pression PSCD ;
- la pression systolique illustrée par la droite 66 (illustrée en pointillés).

Le circuit de traitement 42 détermine ici l'instant de début Tfmd du front montant de la pression PSCD (ou $PSCD_2$), en retranchant la durée T à l'instant Tfdd. On peut également prévoir de rechercher la valeur Tfmd par différents critères dans un intervalle autour de la valeur Tfdd -T. Tfmd respectera par exemple l'inégalité suivante : Tfdd -1,1*T < Tfmd < Tfdd -0,9*T, de préférence Tfdd -1,05*T < Tfmd < Tfdd -0,95*T.

Par exemple :

- si la mesure de ressemblance entre les signaux $PSCP_2$ et $PSCD_2$ est suffisamment élevée (l'écart quadratique moyen est inférieur à un seuil ou si le maximum de la fonction d'auto-corrélation dépasse un seuil), l'instant Tfmd est calculé à partir du pic de la dérivée de $PSCD_2$, qui est le plus rapproché dans le temps de l'instant défini par Tfdd -T. L'instant Tfmd peut être calculé en déterminant la tangente au signal $PSCD_2$ au niveau de ce pic de la dérivée de $PSCD_2$, puis en déterminant l'intersection entre cette tangente et la pression diastolique distale extrapolée;
- si la mesure de ressemblance entre les signaux $PSCP_2$ et $PSCD_2$ est insuffisante (l'écart quadratique moyen est supérieur à un seuil ou si le maximum de la fonction d'auto-corrélation est inférieur à un seuil), une ressemblance suffisante est recherchée à nouveau sur seulement une portion des signaux des pressions $PSCP_2$ et $PSCD_2$ ou de leur dérivées, au milieu de la période T, de préférence sur une portion entre 20% et 80% de l'intervalle T. On calcule la mesure de ressemblance pour différents décalages temporels (par exemple avec des incréments temporels par exemple entre 0,1ms et 0,5ms (correspondant à la période d'échantillonnage), sur une fenêtre de +/-20ms) entre le signal $PSCD_2$ et le signal $PSCP_2$. La mesure de ressemblance maximale est obtenue pour une valeur Tajust. Si une mesure de ressemblance suffisante est obtenue pour cette portion des signaux, avec ce décalage temporel Tajust, l'instant Tfmd est défini exactement par Tfmd=Tfdd -T + Tajust.

- selon un autre mode de calcul, si la mesure de ressemblance entre les signaux $PSCP_2$ et $PSCD_2$ est insuffisante, on récupère le signal de $PSCP_2$ entre l'instant Tfmp et Tfdp, comme illustré aux figures 9 et 10. On réalise une adaptation du signal récupéré à l'échelle du signal $PSCD_2$, comme illustré à la figure 11. L'échelle d'abscisse (axe temporel) du signal $PSCP_2$ récupéré est inchangée. L'échelle des ordonnées du signal $PSCP_2$ récupéré est adaptée pour que son extremum corresponde à la pression systolique distale définie par la droite 66, et pour que son minimum corresponde à la pression diastolique distale extrapolée définie par la droite 68. Le signal $PSCP_2$ récupéré est adapté et inséré en remplacement du signal $PSCD_2$ entre les instants Tfdd - T et Tfdd. Comme illustré à la figure 12, on peut déterminer l'instant Tfmd comme l'intersection entre la pression diastolique distale extrapolée 68 et la tangente 67 au front montant de ce signal $PSCD_2$ inséré. Cette alternative permet de bénéficier de la précision du signal $PSCP_2$ pour déterminer l'instant Tfmd. D'autres modes d'adaptation d'un front montant du signal $PSCP_2$ sur le signal $PSCD_2$ peuvent également être envisagés, en vue de définir l'instant Tfmd.

- si la mesure de ressemblance entre les signaux $PSCP_2$ et $PSCD_2$ est suffisante, si un deuxième pic de la dérivée de la pression $PSCD_2$ est détecté, et si ce deuxième pic de la dérivée de la pression $PSCD_2$ est plus rapproché dans le temps de l'instant défini par (Tfdd -T) que le 1er pic de la dérivée détecté dans le sens croissant du temps, le circuit de traitement 42 pourra générer un signal d'alerte approprié pour attirer l'attention du praticien, ceci pouvant être associé à une sténose coronaire. Toujours dans ce cas, un autre paramètre à prendre en compte pourra être l'amplitude de la pression correspondant au 1er pic de la dérivé de la pression $PSCD_2$ détecté dans le sens croissant du temps et le rapport entre cette amplitude et la valeur de la pression au moment Tqrs juste avant le complexe QRS (voir figure 13): lorsque ce rapport est au-dessus d'un seuil, le circuit de traitement 42 pourra générer un signal d'alerte approprié pour attirer l'attention du praticien, ceci pouvant être associé également à une sténose coronaire.

Lorsque la mesure de ressemblance entre les signaux $PSCP_2$ et $PSCD_2$ est insuffisante, le circuit de traitement 42 pourra générer un signal d'alerte approprié pour attirer l'attention du praticien, ceci pouvant être associé à une sténose coronaire très serrée.

La figure 5 est un diagramme illustrant en superposition un exemple de pression sanguine coronaire proximale PSCP (pour une artère coronaire antérieure descendante gauche) sur plusieurs battements cardiaques, avec un signal d'électrocardiogramme correspondant ECG, la dérivée de la pression PSCP, et un signal proximal SPR recomposé à partir du signal d'électrocardiogramme. Le signal proximal recomposé SPR correspondant à la courbe du bas en position proximale peut être défini selon la relation suivante :

SPR = une constante, une fonction triangulaire ou de préférence l'amplitude du complexe QRS du signal d'électrocardiogramme d'une largeur de préférence de 10-20 ms et centrée sur l'instant Tfmp.
SPR = 0 sinon.

La figure 6 est un diagramme illustrant en superposition un exemple de pression PSCD sur plusieurs battements cardiaques, avec un signal d'électrocardiogramme correspondant ECG, la dérivée de la pression PSCD, et un signal distal SDR recomposé à partir du signal d'électrocardiogramme. Le signal distal recomposé SDR correspondant à la courbe du bas en position distale peut être défini selon la relation suivante :

SDR = une constante, une fonction triangulaire ou de préférence l'amplitude du complexe QRS du signal de l'électrocardiogramme d'une largeur de préférence de 10-20 ms et centrée sur l'instant Tfmd.
SDR = 0 sinon.

Le temps de propagation Tmd de l'onde de pression sanguine dans l'artère coronaire, entre les positions de mesure proximale et distale, est déterminé par la relation : Tmd=(Tfmd-Tfmp) (modulo Tc), c'est-à-dire le reste de la division de Tmd par Tc.

Tc est la période moyenne d'une pulsation sanguine, au cas où le signal PSCP (ou $PSCP_2$) et le signal PSCD (ou $PSCD_2$) utilisés pour les calculs ne sont pas simultanés.

La vitesse d'onde de pouls coronaire CPWV est alors calculée par la relation suivante :

$$CPWV= Dmd/Tmd$$

La vitesse CPWV calculée peut être comparée à un seuil de référence pour une artère et un patient similaires. Lorsque la vitesse CPWV franchit un tel seuil de référence (le cas échéant un seuil bas ou un seuil haut), le circuit de traitement 42 pourra générer un signal d'alerte approprié pour attirer l'attention du praticien. Différents seuils pourront être utilisés, notamment en fonction de différents facteurs de risque, tels que l'hypertension, le diabète, la dyslipidémie, le tabagisme, l'hérédité de problèmes cardiovasculaires coronaires, un épisode cardiovasculaire coronaire antérieur.

Dans le cas où on ne dispose pas des signaux PSCP et PSCD en simultané, les instants Tfmd et Tfmp peuvent être déterminés par leurs décalages respectifs Offsprox et Offsdis par rapport au complexe QRS de l'électrocardiogramme. On calcule par exemple l'auto-corrélation entre un signal d'électrocardiogramme et le signal recomposé SPR à partir de PSCP (ou $PSCP_2$) d'une part, et l'auto-corrélation entre un signal d'électrocardiogramme et le signal recomposé SDR à partir de PSCD (ou $PSCD_2$) d'autre part. Une fonction d'auto-corrélation de paramètre k entre deux fonctions S1 et S2 comprend typiquement le calcul suivant :

$$\sum_{i=1}^{N} S1(i) * S2(i + k)$$

La fonction d'auto-corrélation peut être normalisée. La fonction d'autocorrélation peut également être moyennée sur plusieurs cycles cardiaques pour limiter l'influence d'éventuelles dispersions dues à la respiration conduisant à de fortes erreurs sur un unique cycle.

Avec i un indice temporel d'un échantillon de la fonction S1 ou S2, N un nombre d'échantillons étudiés. En effectuant ce calcul pour différentes valeurs du paramètre k, le maximum de la fonction d'auto-corrélation est obtenu pour la valeur K correspondant au décalage temporel entre les fonctions S1 et S2. Le décalage temporel entre les fonctions S1 et S2 vaut alors K*ΔT, avec ΔT la période d'échantillonnage des fonctions S1 et S2.

Les fonctions d'auto-corrélation permettent donc de calculer le décalage Offsprox entre le complexe QRS de l'électrocardiogramme et le front montant de la PSCP (ou $PSCP_2$) d'une part, et le décalage temporel Offsdis entre le complexe QRS de l'électrocardiogramme et le front montant de la PSCD ($PSCD_2$) d'autre part. On déduit la durée Tmd par Tmd = Offsdis- Offsprox.

S'il est possible de calculer la fonction d'auto-corrélation entre l'électrocardiogramme et les signaux recomposés SPR et SDR sur plusieurs périodes d'analyse consécutives, il sera possible d'obtenir des suites temporelles de décalages Offsprox et respectivement Offsdis. Lorsque le pic de la fonction d'auto-corrélation a une valeur trop basse (au-dessous d'un seuil valant de préférence entre 0.4 et 0.6) sur une période d'analyse, cette période d'analyse sera exclue des suites temporelles des valeurs Offsdis ou Offsprox, car ceci doit correspondre à un signal d'électrocardiogramme parasité ou d'une détection erronée des temps Tfmp et Tfmd.

Dans le cas de périodes d'analyse consécutives, des suites temporelles de décalages Offsprox et respectivement Offsdis peuvent être générées. Les moyennes de ces suites temporelles de décalages $M_{Offsprox}$ et $M_{Offsdis}$ seront utilisées pour calculer la durée Tmd = $M_{Offsdis}$-$M_{Offsprox}$. Lorsqu'une relation linéaire peut être mise en évidence entre les suites temporelles des décalages Offsprox et Offsdis et les suites temporelles des pressions artérielles diastoliques (PAD) correspondantes, moyennées sur les mêmes périodes que lesdits décalages, une correction de ces décalages Offsprox et Offsdis sera effectuée selon ce qui suit afin d'obtenir les décalages pour des pressions artérielles diastoliques constantes:

- une PAD commune PADc sera calculée entre la PAD proximale et la PAD distale: de préférence la moyenne de la PAD proximale et distale. Sinon, on prend l'une des de deux pressions artérielles diastoliques proximale ou distale.
- des régressions linéaires seront recherchées dans les nuages de points Offsprox par rapport à la PAD en position proximale (PADprox) selon la loi: Offsprox = Kprox x PADprox + Cprox. Le coefficient de corrélation Rprox et la probabilité Pprox de la régression seront également calculés.
- des régressions linéaires seront recherchées dans les nuages de points Offsdis par rapport à la PAD en position distale (PADdis) selon la loi: Offsdis = Kdis x PADdis + Cdis. Le coefficient de corrélation Rdis et la probabilité Pdis de la régression seront également calculés.
- Les décalages Offsprox et Offsdis seront corrigés pour extrapoler leur valeur pour cette valeur commune PADc.
- Si des régressions linéaires sont mises en évidence dans les deux positions proximale et distale, c'est-à-dire si Rprox et Rdis sont au-dessus d'un seuil donné et/ou Kprox et Kdis sont au-dessus d'un seuil donné et/ou les probabilités Pprox et Pdis sont au-dessous d'un seuil donné, les décalages corrigés Offsprox' et Offsdis' seront:

  ○

$$Offsprox' = Offsprox - Kprox \times (PADprox - PADc)$$

$$Offsdis' = Offsdis - Kdis \times (PADdis - PADc)$$

- Dans le cas où une régression linéaire est mise en évidence uniquement en position proximale càd Rprox est au-dessus d'un seuil donné et/ou Kprox est au-dessus d'un seuil donné et/ou la probabilité Pprox est au-dessous d'un seuil donné, les décalages corrigés Offsprox' seront:

$$Offsprox' = Offsprox - Kprox \times (PADprox - PADc),$$

avec PADc = PADdis.

- Dans le cas où une régression linéaire est mise en évidence uniquement en position distale, c'est-à-dire si Rdis est au-dessus d'un seuil donné et/ou Kdis est au-dessus d'un seuil donné et/ou la probabilité Pdis est au-dessous d'un seuil donné, les décalages corrigés Offsdis' seront:

$$Offsdis' = Offsdis - Kdis \times (PADdis - PADc),$$

avec PADc = PADprox.

Selon un autre mode de calcul, la correction des décalages pourra se faire de la même manière en fonction des pression artérielles systoliques ou des fréquences cardiaques à la place des pression artérielles diastoliques.

Lorsque les moyennes des suites temporelles des pressions PADprox et PADdis font apparaître une baisse de la pression distale, l'amplitude de la dite baisse étant supérieure à un seuil donné, le circuit de traitement 42 pourra générer un signal d'alerte approprié pour attirer l'attention du praticien, ceci pouvant être associé à une sténose coronaire.

Par ailleurs, le circuit de traitement 42 pourra générer un signal d'alerte pour d'autres cas de figure pouvant être associés à une sténose coronaire. Après avoir déterminé la valeur Tfmd, on calcule la valeur Tpfm=Tfmd-Tana, avec Tana une valeur comprise entre 5 et 10ms. Tana correspond à une durée d'analyse correspondant à une montée d'artéfact de la pression sanguine coronaire distale avant le front montant. On détermine la valeur Ptpfm de PSCD à l'instant Tpfm. On calcule le rapport R=Ptpfm/PDE, avec PDE la pression diastolique extrapolée en position distale. Si R dépasse un seuil (compris entre 0.95 et 1.05 par exemple), le circuit de traitement 42 pourra générer un signal d'alerte, un tel rapport R pouvant être très représentatif du caractère ischémiant d'une sténose coronaire, sans avoir recours à des substances vasodilatatrices comme l'adénosine.

Les procédés de détermination des instants Tfmp et Tfmd décrits peuvent mettre en oeuvre la recherche d'une intersection avec une droite représentative d'une pression diastolique. Or, la pression diastolique peut être sujette à d'importantes variations dans le temps. Il peut donc s'avérer intéressant de réaliser une correction sur les valeurs de pression diastolique mesurées en utilisant un filtre passe-bas pour filtrer les variations rapides dans le signal PSCP ou PSCD (ou $PSCP_2$ et $PSCD_2$).

L'utilisation d'un fil-guide de FFR, dont l'utilisation est validée par les autorités de santé et fait partie de la routine clinique, permet d'utiliser un système selon l'invention avec un processus de validation clinique sensiblement allégé.

Dans l'exemple détaillé précédemment, le fil-guide 3 de FFR comporte deux capteurs de pression 31 et 32 distants d'une distance Dmd prédéterminée fixe. On peut également prévoir d'utiliser un fil-guide de FFR muni d'un unique capteur de pression, déplacé par le praticien d'une distance prédéfinie entre la position distale et la position proximale dans l'artère coronaire. Lors de l'analyse des signaux de mesure respectifs en position proximale et en position distale, cette distance Dmd est prise en compte pour le calcul de la vitesse d'onde de pouls.

On peut par exemple envisager d'échantillonner une pression coronaire distale et/ou une pression coronaire proximale à une fréquence comprise entre 200 Hz et 2 kHz. Pour une fréquence d'échantillonnage jugée insuffisante, on peut procéder à une interpolation des valeurs d'échantillonnage (par exemple par utilisation de splines cubiques), puis à un nouvel échantillonnage du signal interpolé à une fréquence supérieure à la fréquence d'échantillonnage initiale (sur-échantillonnage). Par exemple, pour une fréquence d'échantillonnage de 500 Hz, on peut envisager un sur-échantillonnage du signal interpolé à une fréquence de 2 kHz ou plus.

Des tests ont été réalisés sur un échantillon de 59 patients par un système de détermination de vitesse d'onde de pouls coronaire selon l'invention. Ces tests ont été réalisés sur des patients soumis à des angiogrammes coronaires avec prescription d'une analyse FFR. La prescription d'analyse FFR faisait notamment suite à un examen visuel constatant une réduction de diamètre de plus de 50 % au niveau d'une sténose, au niveau de l'artère coronaire gauche descendante (LDA), de l'artère coronaire droite (RCA) ou de l'artère coronaire circonflexe (Cx).

Les analyses FFR ont été réalisées avec des fils-guides de mesure de pression commercialisés sous la référence commerciale C12058 par la société St. Jude Medical. La pression sanguine invasive a été mesurée par ces fils-guides et échantillonnée sur un ordinateur par l'intermédiaire d'une carte d'acquisition analogique/numérique à une fréquence de 500 Hz. Des signaux d'électrocardiogramme ont été mesurés et échantillonnés en simultané. Des signaux de pression sanguine et d'électrocardiogrammes ont également été mesurés simultanément au niveau du sinus Valsalva, au niveau du tronc artériel brachio-céphalique et au niveau de l'artère radiale. Pour déterminer la distance entre les emplacements de mesure des pressions distale et proximale, cette distance a été déterminée en fonction du déplacement de la partie externe du fil-guide.

En désignant la vitesse d'onde de pouls coronaire par la valeur CPWV, nous avons mesuré une valeur moyenne de CPWV de 10,37 (+/-6,23)m/s pour ces patients avec une excellente reproductibilité. Plus précisément, nous avons mesuré une valeur moyenne de CPWV de 10,06 (+/-4,85)m/s pour les artères coronaires interventriculaires antérieures gauches, une valeur moyenne de CPWV de 10,07 (+/-6, 86)m/s pour les artères coronaires droites, et une valeur moyenne de CPWV de 12,4 (+/-7,06)m/s pour les artères coronaires circonflexes. On a constaté que la valeur CPWV était supérieure pour les artères coronaires comportant un stent 14,25 (+/-5,68)m/s. Par ailleurs pour une même artère coronaire, l'implantation d'un stent entrainait une augmentation de la CPWV de 9,79 (+/-5,1) m/s à 17,34 (+/-9,03)m/s ce qui mécaniquement semble être un élément attendu puisque l'on implante un élément métallique au sein d'une structure élastique. Les déterminants de la CPWV étaient, la fonction rénale, l'indice de masse corporelle, la présence d'un stent.

Par ailleurs nous avons constaté une corrélation significative entre les valeurs de vitesse de l'onde de pouls aortique mesurées de façon invasives et non invasives. Ces constats permettent de conclure à la fiabilité des valeurs CPWV déterminé par un système selon l'invention.

L'invention peut s'avérer particulièrement utile pour la détection de sténoses intermédiaires dans des artères coronaires, de telles sténoses étant généralement indétectables, en particulier chez des jeunes patients.

**Revendications**

**1.** Système de détermination d'une vitesse d'onde de pouls coronaire (4), comprenant :

- une interface de réception (41) d'un signal de pression sanguine proximale dans une artère coronaire (10) et de réception d'un signal de pression sanguine distale dans cette artère coronaire;
- un dispositif de traitement (42), configuré pour :

- identifier un instant Tfmp du début d'un front montant et un instant Tfdp du début d'un front descendant à partir d'un signal de pression proximale reçu ;
- calculer la durée T entre les instants Tfdp et Tfmp identifiés ;
- identifier un instant Tfdd du début du front descendant à partir d'un signal de pression distale reçu ;
- identifier un instant Tfmd du début du front montant du signal de pression distale, après avoir retranché ladite durée calculée T de l'instant Tfdd du début du front descendant du signal de pression distale ;
- récupérer la valeur de la distance entre le lieu de mesure de la pression proximale et le lieu de mesure de la pression distale ;
- calculer la durée séparant le début des fronts montants identifiés respectifs du signal de pression proximale et du signal de pression distale ;
- calculer la vitesse d'onde de pouls coronaire en fonction de ladite durée séparant les fronts montants et de ladite valeur de distance récupérée.

**2.** Système de détermination d'une vitesse d'onde de pouls coronaire selon la revendication 1, dans lequel ledit dispositif de traitement (42) est configuré pour calculer la dérivée du signal de pression sanguine distale, et configuré pour identifier Tfmd comme correspondant à l'instant du pic de la dérivée calculée le plus proche de l'instant défini par Tfdd-T.

3. Système de détermination d'une vitesse d'onde de pouls coronaire selon la revendication 1 ou 2, dans lequel ledit dispositif de traitement (42) est configuré pour :

- dériver un signal de pression sanguine distale $PSCD_2$ par soustraction d'une pression diastolique distale du signal de pression sanguine distale reçu ;
- dériver un signal de pression sanguine proximale $PSCP_2$ par soustraction d'une pression diastolique proximale du signal de pression sanguine proximale reçu ;
- synchroniser les signaux $PSCD_2$ et $PSCP_2$ par le début de leurs fronts descendants respectifs;
- calculer une mesure de ressemblance entre les signaux $PSCD_2$ et $PSCP_2$ sur la durée T.

4. Système de détermination d'une vitesse d'onde de pouls coronaire selon l'une quelconque des revendications 1 à 3, dans lequel ledit dispositif de traitement est configuré pour déterminer Tfmd dans un intervalle défini selon l'inégalité suivante :

$$Tfdd-1,1*T< Tfmd < Tfdd -0,9*T.$$

5. Système de détermination d'une vitesse d'onde de pouls coronaire selon la revendication 1, dans lequel le dispositif de traitement (42) détermine l'instant Tfmd à l'intersection entre une pression diastolique distale et une tangente à un front montant d'un signal de pression distale.

6. Système de détermination d'une vitesse d'onde de pouls coronaire selon l'une quelconque des revendications précédentes, dans lequel l'interface de réception (41) est configurée pour recevoir un signal d'électrocardiogramme, et dans lequel le dispositif de traitement (42) est configuré pour calculer les durées séparant un complexe QRS du signal d'électrocardiogramme respectivement de l'instant Tfmp et de l'instant Tfmd.

7. Système de détermination d'une vitesse d'onde de pouls coronaire selon les revendications 5 et 6, dans lequel le dispositif de traitement (42) détermine ladite pression diastolique distale en calculant l'intersection entre :

- une fonction polynomiale ou logarithmique ajustée au signal de pression distale avant l'instant du début du complexe QRS et extrapolée après l'instant du début du complexe QRS ; et
- la tangente au front montant de la pression distale.

8. Système de détermination d'une vitesse d'onde de pouls coronaire selon l'une quelconque des revendications précédentes, dans lequel le dispositif de traitement (42) détermine l'instant Tfmp à l'intersection entre une tangente au front montant du signal de pression proximale et une pression diastolique proximale, et dans lequel le dispositif de traitement (42) détermine l'instant Tfdp à l'intersection entre une tangente au front descendant du signal de pression proximale et une pression systolique proximale.

9. Système de détermination d'une vitesse d'onde de pouls coronaire selon la revendication 5, dans lequel le dispositif de traitement (42) est configuré pour calculer les durées séparant un complexe QRS du signal d'électrocardiogramme respectivement de l'instant Tfmp et de l'instant Tfmd en:

- créant un premier signal recomposé égal à zéro partout sauf au cours d'une période d'une largeur de 10-20 ms et centrée sur l'instant correspondant au front montant de la pression sanguine Tfmp au cours de laquelle ledit signal recomposé est égal à une constante, à une fonction triangulaire ou à l'amplitude du complexe QRS du signal d'électrocardiogramme,
- créant un deuxième signal recomposé égal à zéro partout sauf au cours d'une période d'une largeur de 10-20 ms et centrée sur l'instant correspondant au front montant de la pression sanguine Tfmd au cours de laquelle ledit signal recomposé est égal à une constante, à une fonction triangulaire ou à l'amplitude du complexe QRS du signal d'électrocardiogramme,
- calculant la fonction d'inter-corrélation entre le signal de l'électrocardiogramme et le premier signal recomposé sur la durée d'un cycle cardiaque ou sur la durée d'un cycle respiratoire ;
- calculant la fonction d'inter-corrélation entre le signal de l'électrocardiogramme et le deuxième signal recomposé sur la durée d'un cycle cardiaque ou sur la durée d'un cycle respiratoire ;
- calculant la moyenne sur un cycle cardiaque ou respiratoire de ladite durée séparant un complexe QRS du signal d'électrocardiogramme respectivement de l'instant Tfmp et de l'instant Tfmd, à partir du décalage entre l'électrocardiogramme et les signaux recomposés pour lesquels la fonction d'inter-corrélation est maximum, si et seulement si la valeur de ce maximum est au-dessus d'un seuil compris de préférence entre 0.4 et 0.6.

10. Système de détermination d'une vitesse d'onde de pouls coronaire selon la revendication 3, dans lequel, si le calcul de la mesure de ressemblance entre les signaux $PSCD_2$ et $PSCP_2$ sur la durée T est insuffisante, le dispositif de traitement (42) calcule une mesure de ressemblance entre les signaux $PSCD_2$ et $PSCP_2$ sur un intervalle compris entre 0,2*T et 0,8*T des signaux synchronisés, avec différentes valeurs de décalages temporels, détermine pour quel décalage temporel Tajust cette mesure de ressemblance entre les signaux $PSCD_2$ et $PSCP_2$ sur cet intervalle est maximale, détermine Tfmd = Tfdd-T+Tajust.

11. Système de détermination d'une vitesse d'onde de pouls coronaire selon la revendication 3, dans lequel, si le calcul de la mesure de ressemblance entre les signaux $PSCD_2$ et $PSCP_2$ sur la durée T est insuffisante, le dispositif de traitement (42) :

   - récupère un signal de pression sanguine proximale entre les instants Tfmp et Tfdp ;
   - adapte le signal récupéré à l'échelle du signal de pression sanguine distale ;
   - remplace le signal de pression sanguine distale entre les instants Tfdd -T et Tfdd par le signal de pression récupéré et adapté;
   - détermine Tfmd comme l'intersection entre la tangente au signal récupéré adapté et une pression diastolique distale.

12. Système de détermination d'une vitesse d'onde de pouls coronaire selon l'une quelconque des revendications précédentes, dans lequel le dispositif de traitement (42) :

   - détermine la valeur du signal de pression sanguine distale à l'instant Tpfm=Tfmd-Tana, avec Tana compris entre 5 et 10ms ;
   - calcule le rapport entre ladite valeur de pression sanguine déterminée à l'instant Tpfm et une pression sanguine diastolique distale ;
   - génère un signal d'alerte si le rapport calculé est supérieur à un seuil compris entre 0.95 et 1.05.

13. Système de détermination d'une vitesse d'onde de pouls coronaire selon l'une quelconque des revendications précédentes, comprenant en outre un fil-guide allongé de FFR, comportant deux capteurs de pression décalés d'une distance prédéfinie sur la longueur du fil-guide, lesdits deux capteurs de pression étant connectés à ladite interface de réception, ladite interface de réception comprenant un circuit d'échantillonnage des signaux respectifs desdits capteurs de pression.

14. Système de détermination d'une vitesse d'onde de pouls coronaire selon la revendication 13, dans lequel ladite distance prédéfinie entre lesdits deux capteurs de pression est comprise entre 70 et 150mm.

**Patentansprüche**

1. System zur Bestimmung einer Geschwindigkeit einer koronaren Pulswelle (4), das enthält:

   - eine Schnittstelle (41) des Empfangs eines proximalen Blutdrucksignals in einer Koronararterie (10) und des Empfangs eines distalen Blutdrucksignals in dieser Koronararterie;
   - eine Verarbeitungsvorrichtung (42), die konfiguriert ist:

      - einen Zeitpunkt Tfmp des Beginns einer ansteigenden Flanke und einen Zeitpunkt Tfdp des Beginns einer abfallenden Flanke ausgehend von einem empfangenen proximalen Drucksignal zu erkennen;
      - die Dauer T zwischen den erkannten Zeitpunkten Tfdp und Tfmp zu berechnen;
      - einen Zeitpunkt Tfdd des Beginns der abfallenden Flanke ausgehend von einem empfangenen distalen Drucksignal zu erkennen;
      - einen Zeitpunkt Tfmd des Beginns der ansteigenden Flanke des distalen Drucksignals zu erkennen, nachdem die berechnete Dauer T vom Zeitpunkt Tfdd des Beginns der abfallenden Flanke des distalen Drucksignals abgezogen wurde;
      - den Wert des Abstands zwischen dem Messort des proximalen Drucks und dem Messort des distalen Drucks abzurufen;

- die den Beginn der erkannten ansteigenden Flanken des proximalen Drucksignals bzw. des distalen Drucksignals trennende Dauer zu berechnen;
- die koronare Pulswellengeschwindigkeit abhängig von der die ansteigenden Flanken trennenden Dauer und dem abgerufenen Abstandswert zu berechnen.

2. System zur Bestimmung einer Geschwindigkeit einer koronaren Pulswelle nach Anspruch 1, wobei die Verarbeitungsvorrichtung (42) konfiguriert ist, die Ableitung des distalen Blutdrucksignals zu berechnen, und konfiguriert ist, Tfmd als dem Zeitpunkt der Spitze der berechneten Ableitung entsprechend zu erkennen, der dem durch Tfdd-T definierten Zeitpunkt am nächsten liegt.

3. System zur Bestimmung einer Geschwindigkeit einer koronaren Pulswelle nach Anspruch 1 oder 2, wobei die Verarbeitungsvorrichtung (42) konfiguriert ist:

- ein distales Blutdrucksignal $PSCD_2$ durch Subtraktion eines distalen diastolischen Drucks vom empfangenen distalen Blutdrucksignal abzuleiten;
- ein proximales Blutdrucksignal $PSCP_2$ durch Subtraktion eines proximalen diastolischen Drucks vom empfangenen proximalen Blutdrucksignal abzuleiten;
- die Signale $PSCD_2$ und $PSCP_2$ durch den Beginn ihrer jeweiligen abfallenden Flanken zu synchronisieren;
- einen Ähnlichkeitsmesswert zwischen den Signalen $PSCD_2$ und $PSCP_2$ über die Dauer T zu berechnen.

4. System zur Bestimmung einer Geschwindigkeit einer koronaren Pulswelle nach einem der Ansprüche 1 bis 3, wobei die Verarbeitungsvorrichtung konfiguriert ist, Tfmd in einem gemäß der folgenden Ungleichheit definierten Zeitabstand zu bestimmen:

$$Tfdd-1,1*T < Tfmd < Tfdd-0,9*T.$$

5. System zur Bestimmung einer Geschwindigkeit einer koronaren Pulswelle nach Anspruch 1, wobei die Verarbeitungsvorrichtung (42) den Zeitpunkt Tfmd am Schnittpunkt zwischen einem distalen diastolischen Druck und einer Tangente an einer ansteigenden Flanke eines distalen Drucksignals bestimmt.

6. System zur Bestimmung einer Geschwindigkeit einer koronaren Pulswelle nach einem der vorhergehenden Ansprüche, wobei die Empfangsschnittstelle (41) konfiguriert ist, ein Elektrokardiogrammsignal zu empfangen, und wobei die Verarbeitungsvorrichtung (42) konfiguriert ist, die Dauern zu berechnen, die einen QRS-Komplex des Elektrokardiogrammsignals vom Zeitpunkt Tfmp bzw. vom Zeitpunkt Tfmd trennen.

7. System zur Bestimmung einer Geschwindigkeit einer koronaren Pulswelle nach den Ansprüchen 5 und 6, wobei die Verarbeitungsvorrichtung (42) den distalen diastolischen Druck bestimmt, indem sie den Schnittpunkt berechnet zwischen:

- einer polynomialen oder logarithmischen Funktion angepasst an das distale Drucksignal vor dem Zeitpunkt des Beginns des QRS-Komplexes und extrapoliert nach dem Zeitpunkt des Beginns des QRS-Komplexes; und
- der Tangente an der ansteigenden Flanke des distalen Drucks.

8. System zur Bestimmung einer Geschwindigkeit einer koronaren Pulswelle nach einem der vorhergehenden Ansprüche, wobei die Verarbeitungsvorrichtung (42) den Zeitpunkt Tfmp am Schnittpunkt zwischen einer Tangente an der ansteigenden Flanke des proximalen Drucksignals und einem proximalen diastolischen Druck bestimmt, und wobei die Verarbeitungsvorrichtung (42) den Zeitpunkt Tfdp am Schnittpunkt zwischen einer Tangente an der abfallenden Flanke des proximalen Drucksignals und einem proximalen systolischen Druck bestimmt.

9. System zur Bestimmung einer Geschwindigkeit einer koronaren Pulswelle nach Anspruch 5, wobei die Verarbeitungsvorrichtung (42) konfiguriert ist, die Dauern zu berechnen, die einen QRS-Komplex des Elektrokardiogrammsignals vom Zeitpunkt Tfmp bzw. vom Zeitpunkt Tfmd trennen, durch:

- Erzeugen eines ersten neu zusammengesetzten Signals gleich Null überall außer während einer Periode einer Breite von 10-20 ms und zentriert auf den Zeitpunkt entsprechend der ansteigenden Flanke des Blutdrucks Tfmp, während der das neu zusammengesetzte Signal gleich einer Konstanten, einer Dreiecksfunktion oder der Amplitude des QRS-Komplexes des Elektrokardiogrammsignals ist,

- Erzeugen eines zweiten neu zusammengesetzten Signals gleich Null überall außer während einer Periode einer Breite von 10-20 ms und zentriert auf den Zeitpunkt entsprechend der ansteigenden Flanke des Blutdrucks Tfmd, während der das neu zusammengesetzte Signal gleich einer Konstanten, einer Dreiecksfunktion oder der Amplitude des QRS-Komplex des Elektrokardiogrammsignals ist,

- Berechnen der Interkorrelationsfunktion zwischen dem Signal des Elektrokardiogramms und dem ersten neu zusammengesetzten Signal über die Dauer eines Herzzyklus oder über die Dauer eines Atemzyklus;

- Berechnen der Interkorrelationsfunktion zwischen dem Signal des Elektrokardiogramms und dem zweiten neu zusammengesetzten Signal über die Dauer eines Herzzyklus oder über die Dauer eines Atemzyklus;

- Berechnen des Mittelwerts über einen Herz- oder Atemzyklus der Dauer, die einen QRS-Komplex des Elektrokardiogrammsignals vom Zeitpunkt Tfmp bzw. vom Zeitpunkt Tfmd trennt, ausgehend von der Verzögerung zwischen dem Elektrokardiogramm und den neu zusammengesetzten Signalen, für die die Interkorrelationsfunktion maximal ist, wenn und nur wenn der Wert dieses Maximums über einer Schwelle ist, die vorzugsweise zwischen 0,4 und 0,6 liegt.

10. System zur Bestimmung einer Geschwindigkeit einer koronaren Pulswelle nach Anspruch 3, wobei, wenn die Berechnung des Ähnlichkeitsmesswerts zwischen den Signalen $PSCD_2$ und $PSCP_2$ über die Dauer T unzureichend ist, die Verarbeitungsvorrichtung (42) einen Ähnlichkeitsmesswert zwischen den Signalen $PSCD_2$ und $PSCP_2$ über einen Zeitabstand zwischen 0,2*T und 0,8*T der synchronisierten Signale berechnet, mit verschiedenen Werten zeitlicher Verzögerungen, bestimmt, für welche zeitliche Verzögerung Tajust dieser Ähnlichkeitsmesswert zwischen den Signalen $PSCD_2$ und $PSCP_2$ über diesen Zeitabstand maximal ist, bestimmt Tfmd = Tfdd-T + Tajust.

11. System zur Bestimmung einer Geschwindigkeit einer koronaren Pulswelle nach Anspruch 3, wobei, wenn die Berechnung des Ähnlichkeitsmesswerts zwischen den Signalen $PSCD_2$ und $PSCP_2$ über die Dauer T unzureichend ist, die Verarbeitungsvorrichtung (42):

- ein proximales Blutdrucksignal zwischen den Zeitpunkten Tfmp und Tfdp abruft;
- das abgerufene Signal an den Maßstab des distalen Blutdrucksignals anpasst;
- das distale Blutdrucksignal zwischen den Zeitpunkten Tfdd-T und Tfdd durch das abgerufene und angepasste Drucksignal ersetzt;
- Tfmd als Schnittpunkt zwischen der Tangente am abgerufenen und angepassten Signal und einem distalen diastolischen Druck bestimmt.

12. System zur Bestimmung einer Geschwindigkeit einer koronaren Pulswelle nach einem der vorhergehenden Ansprüche, wobei die Verarbeitungsvorrichtung (42):

- den Wert des distalen Blutdrucksignals zum Zeitpunkt Tfmp=Tfmd-Tana bestimmt, mit Tana zwischen 5 und 10ms;
- das Verhältnis zwischen dem bestimmten Blutdruckwert zum Zeitpunkt Tfmp und einem distalen diastolischen Blutdruck berechnet;
- ein Warnsignal erzeugt, wenn das berechnete Verhältnis höher als eine Schwelle zwischen 0,95 und 1,05 ist.

13. System zur Bestimmung einer Geschwindigkeit einer koronaren Pulswelle nach einem der vorhergehenden Ansprüche, das außerdem einen länglichen FFR-Führungsdraht enthält, der zwei um einen vordefinierten Abstand über die Länge des Führungsdrahts versetzte Drucksensoren aufweist, wobei die zwei Drucksensoren mit der Empfangsschnittstelle verbunden sind, wobei die Empfangsschnittstelle einen Schaltkreis zur Abtastung der jeweiligen Signale der Drucksensoren enthält.

14. System zur Bestimmung einer Geschwindigkeit einer koronaren Pulswelle nach Anspruch 13, wobei der vordefinierte Abstand zwischen den zwei Drucksensoren zwischen 70 und 150mm liegt.

**Claims**

1. System for determining a coronary pulse wave velocity (4), comprising:

- an interface (41) for receiving a signal of proximal blood pressure in a coronary artery (10) and for receiving a signal of distal blood pressure in said coronary artery;

- a processing device (42), configured to:

- on the basis of a received signal of proximal pressure, identify a time Tfmp of the start of a rising edge and a time Tfdp of the start of a falling edge;
- calculate the duration T between the identified times Tfdp and Tfmp;
- on the basis of a received signal of distal pressure, identify a time Tfdd of the start of the falling edge;
- after having subtracted said calculated duration T from the time Tfdd of the start of the falling edge of the signal of distal pressure, identify a time Tfmd of the start of the rising edge of the signal of distal pressure;
- collect the value of the distance between the place of measurement of the proximal pressure and the place of measurement of the distal pressure;
- calculate the duration separating the start of the respective identified rising edges of the signal of proximal pressure and of the signal of distal pressure;
- calculate the coronary pulse wave velocity depending on said duration separating the rising edges and said collected distance value.

2. System for determining a coronary pulse wave velocity according to Claim 1, wherein said processing device (42) is configured to calculate the derivative of the signal of distal blood pressure, and configured to identify Tfmd as corresponding to the time of the peak of the calculated derivative closest to the time defined by Tfdd-T.

3. System for determining a coronary pulse wave velocity according to Claim 1 or 2, wherein said processing device (42) is configured to:

- derive a signal of distal blood pressure $PSCD_2$ by subtracting a distal diastolic pressure from the received signal of distal blood pressure;
- derive a signal of proximal blood pressure $PSCP_2$ by subtracting a proximal diastolic pressure from the received signal of proximal blood pressure;
- synchronize the signals $PSCD_2$ and $PSCP_2$ using the start of their respective falling edges;
- calculate a measure of the resemblance between the signals $PSCD_2$ and $PSCP_2$ over the duration T.

4. System for determining a coronary pulse wave velocity according to any one of Claims 1 to 3, wherein said processing device is configured to determine Tfmd in an interval defined by the following inequality:

$$Tfdd\text{-}1.1*T < Tfmd < Tfdd\text{-}0.9*T.$$

5. System for determining a coronary pulse wave velocity according to Claim 1, wherein the processing device (42) determines the time Tfmd at the intersection between a distal diastolic pressure and a tangent to a rising edge of a signal of distal pressure.

6. System for determining a coronary pulse wave velocity according to any one of the preceding claims, wherein the receiving interface (41) is configured to receive an electrocardiogram signal, and wherein the processing device (42) is configured to calculate the durations separating a QRS complex of the electrocardiogram signal from the time Tfmp and from the time Tfmd, respectively.

7. System for determining a coronary pulse wave velocity according to Claims 5 and 6, wherein the processing device (42) determines said distal diastolic pressure by calculating the intersection between:

- a logarithmic or polynomial function adjusted to the signal of distal pressure before the time of the start of the QRS complex and extrapolated after the time of the start of the QRS complex; and
- the tangent to the rising edge of the distal pressure.

8. System for determining a coronary pulse wave velocity according to any one of the preceding claims, wherein the processing device (42) determines the time Tfmp at the intersection between a tangent to the rising edge of the signal of proximal pressure and a proximal diastolic pressure, and wherein the processing device (42) determines the time Tfdp at the intersection between a tangent to the falling edge of the signal of proximal pressure and a proximal systolic pressure.

9. System for determining a coronary pulse wave velocity according to Claim 5, wherein the processing device (42) is configured to calculate the durations separating a QRS complex of the electrocardiogram signal from the time Tfmp and from the time Tfmd, respectively, by:

    - creating a first reconstructed signal that is equal to zero everywhere except during a period of width of 10-20 ms, which period is centred on the time Tfmp corresponding to the rising edge of the blood pressure and during which period said reconstructed signal is equal to a constant, to a triangular function or to the amplitude of the QRS complex of the electrocardiogram signal,
    - creating a second reconstructed signal that is equal to zero everywhere except during a period of width of 10-20 ms, which period is centred on the time Tfmd corresponding to the rising edge of the blood pressure and during which period said reconstructed signal is equal to a constant, to a triangular function or to the amplitude of the QRS complex of the electrocardiogram signal,
    - calculating the inter-correlation function of the electrocardiogram signal and of the first reconstructed signal over the duration of one cardiac cycle or over the duration of one respiratory cycle,
    - calculating the inter-correlation function of the electrocardiogram signal and of the second reconstructed signal over the duration of one cardiac cycle or over the duration of one respiratory cycle,
    - on the basis of the offset between the electrocardiogram and the reconstructed signals for which the inter-correlation function is maximum, if and only if the value of this maximum is above a threshold preferably comprised between 0.4 and 0.6, calculating the average over one respiratory or cardiac cycle of said duration separating a QRS complex of the electrocardiogram signal from the time Tfmp and from the time Tfmd, respectively.

10. System for determining a coronary pulse wave velocity according to Claim 3, wherein, if the calculation of the measure of the resemblance between the signals $PSCD_2$ and $PSCP_2$ over the duration T is insufficient, the processing device (42) calculates a measure of the resemblance between the signals $PSCD_2$ and $PSCP_2$ over an interval comprised between 0.2*T and 0.8*T of the synchronized signals, with various temporal offset values, determines for which temporal offset Tajust this measure of the resemblance between the signals $PSCD_2$ and $PSCP_2$ over this interval is maximal, and determines Tfmd = Tfdd-T+Tajust.

11. System for determining a coronary pulse wave velocity according to Claim 3, wherein, if the calculation of the measure of the resemblance between the signals $PSCD_2$ and $PSCP_2$ over the duration T is insufficient, the processing device (42):

    - collects a signal of proximal blood pressure between the times Tfmp and Tfdp;
    - scales the collected signal to the scale of the signal of distal blood pressure;
    - replaces the signal of distal blood pressure between the times Tfdd-T and Tfdd with the collected and scaled signal of pressure;
    - determines Tfmd as the intersection between the tangent to the scaled collected signal and a distal diastolic pressure.

12. System for determining a coronary pulse wave velocity according to any one of the preceding claims, wherein the processing device (42):

    - determines the value of the signal of distal blood pressure at the time Tfmp=Tfmd-Tana, with Tana comprised between 5 and 10 ms;
    - calculates the ratio between said blood-pressure value determined at the time Tfmp and a distal diastolic blood pressure;
    - generates a warning signal if the calculated ratio is higher than a threshold comprised between 0.95 and 1.05.

13. System for determining a coronary pulse wave velocity according to any one of the preceding claims, furthermore comprising an elongate FFR guidewire including two pressure sensors that are offset by a predefined distance along the length of the guidewire, said two pressure sensors being connected to said receiving interface, said receiving interface comprising a circuit for sampling the respective signals of said pressure sensors.

14. System for determining a coronary pulse wave velocity according to Claim 13, wherein said predefined distance between said two pressure sensors is comprised between 70 and 150 mm.

Fig. 1

Fig. 2

Fig. 3

31 30 330 32
35 311
321

39

34 33

33 311 321
38
39
34 36 37

Fig. 4

5

4
42 43

41

Fig. 5

Fig. 6

Fig. 7

Fig. 8

Fig. 9

Fig. 10

Fig. 11

Fig. 12

Fig. 13

## RÉFÉRENCES CITÉES DANS LA DESCRIPTION

**Littérature non-brevet citée dans la description**

- A Coronary Pulse Wave Velocity Measurement System. Proceedings of the 29th Annual International Conférence of the IEEE EMBS. Messieurs Taewoo Nam et alias, 23 Août 2007, 975-977

- Alterations of pressure waveforms along the coronary arteries and the effect of microcirculatory vasodilation. International Journal of Cardiology. Mrs Vavuranakis et alias, 2007, vol. 117, 254-259